# EUROPEAN PATENT APPLICATION

(11) **EP 3 034 125 A1**
(43) Date of publication of application: **22.06.2016**
(21) Application number: 14836492.0
(22) Date of filing: 13.08.2014
(51) Int. Cl.: A61M 25/06

(54) **INTRODUCTION ASSISTANCE INSTRUMENT WITH SIDE HOLE**

(30) Priority: 13.08.2013 JP 2013168003
(71) Applicant: Katera Company, Limited, Tokyo 111-0032 (JP)
(72) Inventor: TAKAHASHI, Yasuhiro, Tokyo 112-0014 (JP); OKADA, Hirotaka, Tokyo 111-0032 (JP)
(74) Representative: Beyer, Andreas
(86) International application number: PCT/JP2014/071365
(87) International publication number: WO 2015/022972

(57) **Abstract**

An introduction assistance instrument with a side hole (11) includes a tubular body (13) adapted to be inserted into a tubular organ of a human body. A side hole (21) is formed in a peripheral wall of the tubular body (13) so as to allow a medical instrument to be introduced into the tubular organ through the side hole (21) of the tubular body (13). Further, there is provided at a tip portion of the tubular body (13) a backflow restriction mechanism configured to restrict backflow of a body fluid in the tubular organ from a tip opening of the tubular body (13) to the side hole (21). The backflow restriction mechanism may be a non-return valve provided between the tip opening of the tubular body (13) and the side hole (21) in an inner lumen of the tubular body (13) or a tip member provided at the tip of the tubular body (13) and having a through hole formed at a central portion thereof.

## Description

### Technical Field

The present invention relates to an introduction assistance instrument which includes a tubular body adapted to be inserted into a tubular organ of a human body and which allows a medical instrument to be introduced into the tubular organ through the tubular body.

### Background Art

In a surgical operation inside a tubular organ of a human body, such as a blood vessel, an introduction assistance instrument such as a sheath introducer disclosed in PTL1 or a guide catheter disclosed in PTL2 is inserted into the tubular organ and placed in position, and then a treatment instrument such as a treatment catheter is introduced to a lesion location through an inner lumen of the introduction assistance instrument.

When introducing the treatment instrument to the lesion location through the introduction assistance instrument placed in the tubular organ, a treatment instrument guide wire is first inserted into the inner lumen of the introduction assistance instrument placed in the tubular organ so that its tip reaches the vicinity of the lesion location, and then the tip of the treatment instrument such as the treatment catheter is inserted to a base end portion of the treatment instrument guide wire and guided to the lesion location along the treatment instrument guide wire. In this process, the treatment instrument guide wire or the treatment instrument is advanced forward, by transmitting a pushing force, given from the base end side (doctor's hand manipulation side) of the treatment instrument guide wire or the treatment instrument, to its tip portion.

### Citation List

### Patent Literatures

PTL1: Japanese Unexamined Patent Publication No. 2003-325671
PTL2: Japanese Unexamined Patent Publication No. 2009-22432
PTL3: Japanese Unexamined Patent Publication No. 2000-102618

### Summary of Invention

### Technical Problem

When a lesion location is in a side branch vessel bifurcating from a vessel into which the introduction assistance instrument is introduced (hereinafter referred to as an "introduction vessel"), the treatment instrument guide wire protruding from the tip of the introduction assistance instrument needs to be bent and inserted into the side branch vessel and likewise the treatment instrument also needs to be bent and inserted into the side branch vessel. However, in the state where the treatment instrument guide wire or the treatment instrument (hereinafter referred to as the guide wire or the like) is bent outside the introduction assistance instrument, it is difficult to transmit a pushing force for advancing the guide wire or the like to the tip portion of the guide wire or the like. Particularly, if the side branch vessel has a narrowed part, the guide wire or the like receives a reaction force when the tip portion of the guide wire or the like passes through the narrowed part. If the guide wire or the like receives such a reaction force, the direction of the pushing force and the direction of the reaction force do not coincide with each other in the state where the guide wire or the like is bent, so that the guide wire or the like deflects laterally near its bent portion. Therefore, it becomes more difficult to transmit the pushing force for advancing the guide wire or the like to the tip portion of the guide wire or the like.

PTL3 proposes, as an introduction assistance instrument that facilitates an approach to such a side branch vessel, a sheath having at least one side hole formed in a surface of a sheath tube thereof. However, the introduction assistance instrument disclosed in PTL3 is formed with the side hole, and therefore allows to flow out through the side hole when the tip of the introduction assistance instrument is inserted into an introduction vessel so that blood in the introduction vessel enters from a tip opening of the introduction assistance instrument. As a result, there arises a problem that when the tip of the introduction assistance instrument is inserted into the introduction vessel and the side hole is located in a tissue outside the vessel (e.g., a subcutaneous tissue), the blood in the introduction vessel is allowed to flow out to the tissue outside the vessel.

Accordingly, it is an object of the present invention to solve the problems existing in the prior art and to inhibit, when using an introduction assistance instrument with a side hole, backflow of a body fluid in a tubular organ of a human body from a tip opening to the side hole of the introduction assistance instrument.

### Solution to Problem

In view of the above object, the present invention provides an introduction assistance instrument with a side hole, including a tubular body adapted to be inserted into a tubular organ of a human body, the side hole formed in a peripheral wall of the tubular body so as to allow a medical instrument to be introduced into the tubular organ through the side hole of the tubular body, wherein the introduction assistance instrument includes a backflow restriction mechanism provided at a tip portion of the tubular body, the backflow restriction mechanism restricting backflow of a body fluid in the tubular organ from a tip opening of the tubular body to the side hole.

The above-mentioned introduction assistance instrument includes the backflow restriction mechanism at the tip portion of the tubular body, and therefore the body fluid in the tubular organ into which the introduction assistance instrument is inserted is inhibited from flowing back from the tip opening of the tubular body to the side hole.

In one embodiment, the backflow restriction mechanism may be a non-return valve provided between the tip opening of the tubular body and the side hole in an inner lumen of the tubular body.

In another embodiment, the backflow restriction mechanism may be a tip member provided at a tip of the tubular body and shaped so that an outer peripheral surface thereof tapers toward a tip side thereof, the tip member formed at a center portion thereof with a through hole that allows passage of a guide wire. In this case, a cross section of a distal end portion of the through hole is preferably smaller than a cross section of a proximal end portion of the through hole.

The side hole has a peripheral edge including a distal end portion formed so as to have a semi-circular shape when projected onto a plane and a proximal end portion formed so as to have a semi-elliptical shape when projected onto the plane.

The introduction assistance instrument may be, for example, a sheath introducer, a guide catheter, or a catheter for penetrating a stenotic lesion.

### Advantageous Effects of Invention

According to the introduction assistance instrument of the present invention, a body fluid in a tubular organ, into which the introduction assistance instrument is inserted, (hereinafter referred to as the introduction tubular organ) is inhibited from flowing back from the tip opening to the side hole of the tubular body. Therefore, even when the tip of the introduction assistance instrument is inserted into the introduction tubular organ and the side hole is located in a tissue outside the introduction tubular organ (e.g. a subcutaneous tissue), it is possible to inhibit the body fluid in the introduction tubular organ from flowing out to the tissue outside the introduction tubular organ.

### Brief Description of Drawings

[FIG. 1] FIG.1 is a perspective view showing an overall configuration diagram of an introduction assistance instrument with a side hole according to a first embodiment of the present invention.
[FIG. 2A] FIG.2A is a diagram in which the side hole of the introduction assistance instrument is seen in a direction perpendicular to a center axis of a tubular body of the introduction assistance instrument.
[FIG. 2B] FIG. 2B is a diagram in which the side hole of the introduction assistance instrument is seen in the direction perpendicular to the center axis of the tubular body.
[FIG. 3] FIG. 3 is an explanatory diagram showing a state when a pushing force and a reaction force act simultaneously on a guide wire extending out from the tip of an introduction assistance instrument and further extending while bending.
[FIG. 4] FIG. 4 is an explanatory diagram showing a state when a pushing force and a reaction force act simultaneously on a guide wire extending out from the side hole of the introduction assistance instrument with side hole and further extending while bending.
[FIG. 5] FIG. 5 is a first explanatory diagram for explaining a method of designing the shape of the side hole of the introduction assistance instrument.
[FIG. 6] FIG. 6 is a second explanatory diagram for explaining the method of designing the shape of the side hole of the introduction assistance instrument.
[FIG. 7] FIG. 7 is a perspective view showing, on an enlarged scale, a tip portion of the introduction assistance instrument with the side hole in which a non-return valve is provided.
[FIG. 8A] FIG. 8A is a perspective view showing a first embodiment of the non-return valve.
[FIG. 8B] FIG. 8B is a sectional view showing a variation of the non-return valve.
[FIG. 8C] FIG. 8C is a sectional view showing a variation of the non-return valve.
[FIG. 8D] FIG. 8D is a sectional view showing a variation of the non-return valve.
[FIG. 9] FIG. 9 is a perspective view showing a second embodiment of the non-return valve.
[FIG. 10A] FIG. 10 is a perspective view showing a third embodiment of the non-return valve.
[FIG. 10B] FIG. 10B is a top view of the non-return valve shown in FIG. 10A.
[FIG. 11A] FIG. 11A is a top view showing a variation of the third embodiment of the non-return valve shown in FIGS. 10A and 10B.
[FIG. 11B] FIG. 11B is a top view showing a variation of the third embodiment of the non-return valve shown in FIGS. 10A and 10B.
[FIG. 12A] FIG. 12A is an explanatory diagram for a method of using the introduction assistance instrument with the side hole according to the first embodiment of the present invention.
[FIG. 12B] FIG. 12B is an explanatory diagram for the method of using the introduction assistance instrument with the side hole according to the first embodiment of the present invention.
[FIG. 12C] FIG. 12C is an explanatory diagram for the method of using the introduction assistance instrument with the side hole according to the first embodiment of the present invention.
[FIG. 13A] FIG. 13A is a perspective view showing an introduction assistance instrument with a side hole having a tip member provided at a tip of a tubular body, according to a second embodiment of the present invention.
[FIG. 13B] FIG. 13B is a perspective view showing the introduction assistance instrument with the side hole having the tip member provided at the tip of the tubular body, according to the second embodiment of the present invention.

### Description of Embodiments

Several embodiments of an introduction assistance instrument with a side hole according to the present invention will be described below with reference to the accompanying drawings.

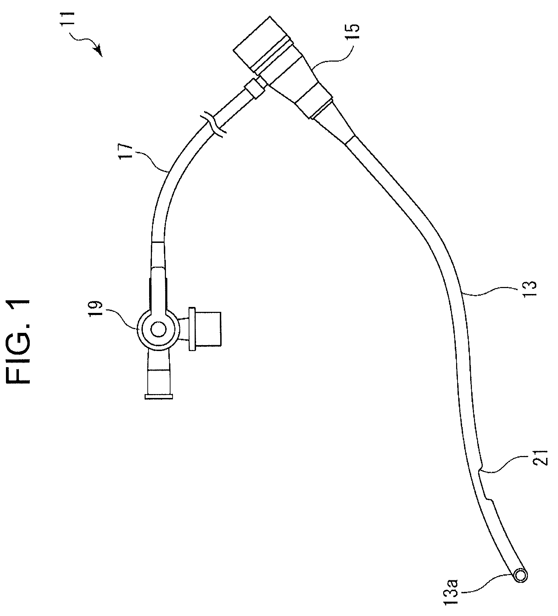

First, referring to FIG. 1, an overall configuration of an introduction assistance instrument 11 with a side hole according to a first embodiment of the present invention will be described. The introduction assistance instrument 11 is an instrument for facilitating introduction of a medical instrument such as a guide wire or a treatment instrument into a tubular organ of a human body, such as a blood vessel. Here, the introduction assistance instrument 11 will be described using a sheath introducer as an example. However, the introduction assistance instrument 11 is not limited to the sheath introducer as long as it is an instrument for assisting introduction of a medical instrument into a tubular organ of a human body, and can also be, for example, a guide catheter, a catheter for penetrating a stenotic lesion, or the like.

The introduction assistance instrument 11 includes a tubular body 13 extending along an axis, an instrument body 15, a connecting tube 17 extending from the instrument body 15, and a three-way switching valve 19. The tubular body 13 is a flexible tube such as a sheath tube formed of a suitable synthetic resin material. The instrument body 15 is a generally cylindrical member such as a sheath hub formed of a suitable synthetic resin material and is fixedly fitted around a base end portion of the tubular body 13. A non-return valve (not shown) is provided at a base end portion (an end portion on the side opposite to an end portion connected to the tubular body 13) of the instrument body 15. The non-return valve is configured to allow insertion of a medical instrument into the instrument body 15 in a liquid-tight state and to be in a closed state after removal of the medical instrument so as to prevent leakage of a liquid or the like from the inside of the instrument body 15 to the outside. Since such a non-return valve is well known and is unrelated to the essential feature of the present invention, a detailed description of its structure will be omitted. The connecting tube 17 is a flexible tube formed of a suitable synthetic resin material and is connected to the instrument body 15 so as to be in communication with the inside of the instrument body 15. The three-way switching valve 19 is connected to a free end portion (an end portion on the side opposite to an end portion connected to the instrument body 15) of the connecting tube 17 and is configured to allow any two of three ports to be in communication with each other by an operation of a lever. Since such a three-way switching valve is well known and is unrelated to the essential feature of the present invention, a detailed description of its structure will be omitted.

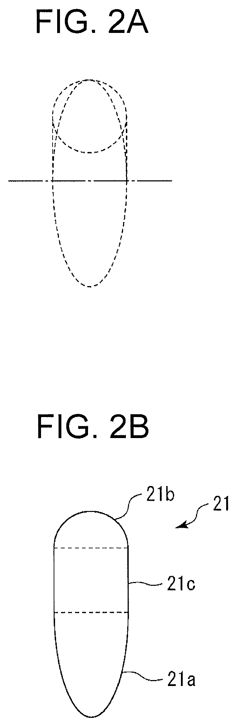

A side hole 21 is formed in a peripheral wall of the tubular body 13, preferably on an distal end (tip portion) side thereof. As shown in FIGS. 2A and 2B, when the side hole 21 is seen from the front in a direction perpendicular to a center axis of the tubular body 13, the side hole 21 has a shape such that a proximal end portion 21a located on the proximal side (the side of the instrument body 15) and having a semi-elliptical shape and a distal end portion 21b located on the distal side (the side of the tip portion 15a) and having a semi-circular shape are connected to each other via a rectangular portion 21c. That is, the side hole 21 is formed by removing the peripheral wall of the tubular body 13 so that a peripheral edge of the side hole 21 has a shape including the proximal end portion 21a formed so as to have a semi-elliptical shape when projected onto a plane in the direction perpendicular to the center axis of the tubular body 13, the distal end portion 21b formed so as to have a semi-circular shape when projected onto the plane in the direction perpendicular to the center axis of the tubular body 13, and the straight portion 21c connecting between the proximal end portion 21a and the distal end portion 21b. It is noted that the semi-elliptical shape means a shape obtained by bisecting an ellipse along a minor axis thereof. The straight portion 21c of the peripheral edge of the side hole 21 is not essential, and the peripheral edge of the side hole 21 may have a shape such that the semi-elliptical proximal end portion 21a and the semi-circular distal end portion 21 are directly connected to each other.

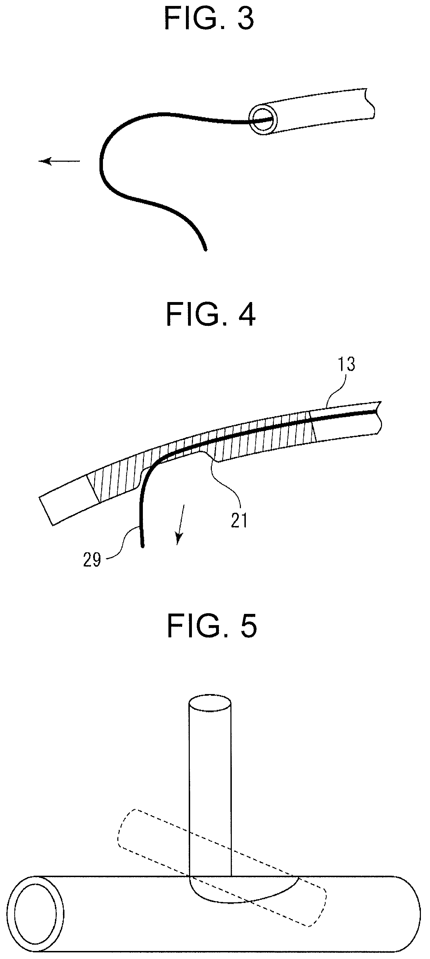

When, for example, introducing a medical instrument (such as a guide wire or a treatment instrument) through the introduction assistance instrument into a side branch vessel bifurcating obliquely at an angle from an introduction vessel into which the introduction assistance instrument is introduced, it is necessary to bend the medical instrument. Further, when passing the medical instrument through a narrowed part of the vessel, a pushing force and, at the same time, a reaction force from a tip portion act on the medical instrument. Thus, when passing the medical instrument through the narrowed part of the side branch vessel, the pushing force and the reaction force simultaneously act on the bent medical instrument. However, since the direction of the pushing force and the direction of the reaction force do not coincide with each other (i.e., are not aligned with each other), the medical instrument tends to deflect to the lateral distal side near the bent portion thereof. Accordingly, when the pushing force and the reaction force act on the medical instrument in the state where the medical instrument is bent after protruding from a tip opening of the tubular body 13 of the introduction assistance instrument, the medical instrument deflects in an extending direction of the introduction vessel as shown by an arrow in FIG. 3 so that the pushing force cannot be efficiently transmitted to the tip portion of the medical instrument, leading to a possibility that time is required for passing through the narrowed part. On the other hand, when the medical instrument is made to approach the side branch vessel through the side hole 21 of the introduction assistance instrument 11, even if the pushing force and, at the same time, the reaction force from the tip portion act on the medical instrument, the medical instrument is, as shown in FIG. 4, pressed against the peripheral wall of the introduction assistance instrument 11 and the peripheral edge of the distal end portion of the side hole 21, so that a backup function is provided to inhibit lateral deflection of the medical instrument by the peripheral wall of the introduction assistance instrument 11 and the peripheral edge of the side hole 21.

Here, a method of designing the shape of the side hole 21 and its action will be described in detail.

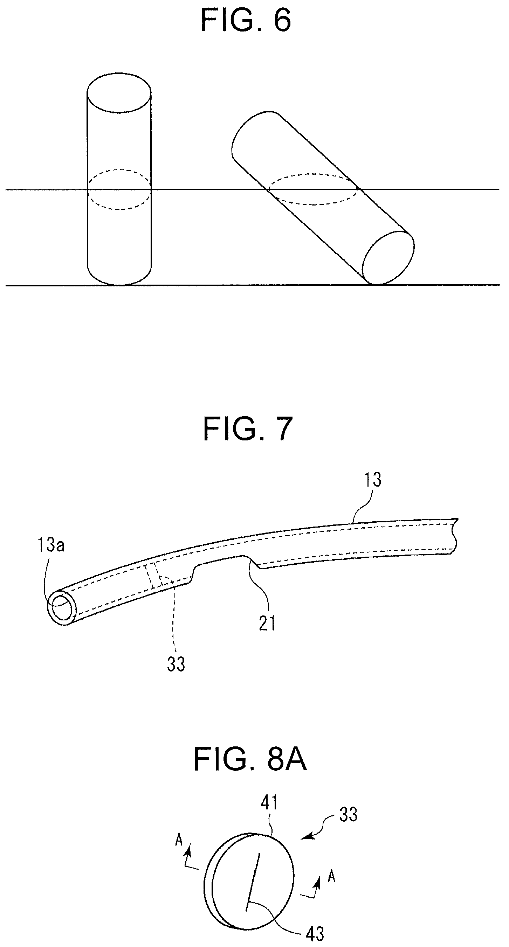

When extending out a medical instrument such as a treatment instrument, extending in an inner lumen of the tubular body 13 and having a generally circular contour in cross section, to the outside through the side hole 21 of the tubular body 13, the medical instrument can be approximated to a cylinder oblique to the center axis of the tubular body 13 in the vicinity of the side hole 21 as shown by a dotted line in FIG. 5. At this time, when a line of intersection between an outer peripheral surface of the cylinder and the tubular body 13 is seen from the front in the direction perpendicular to the center axis of the tubular body 13, the intersection line has an elliptical shape as shown by a dotted line on the right side of FIG. 6. Therefore, if the proximal end portion 21a of the peripheral edge of the side hole 21 is formed so as to have the semi-elliptical shape when projected onto the plane in the direction perpendicular to the center axis of the tubular body 13, it is possible to smoothly pass the medical instrument through the side hole 21 of the tubular body 13 obliquely to the center axis of the tubular body 13. When passing through a narrowed part or the like, the medical instrument attempts to deflect laterally and is pressed against the peripheral edge on the distal side of the side hole 21 as described above so as to extend in a direction generally perpendicular to the center axis of the tubular body 13 so that, as shown by a solid line in FIG. 5, the medical instrument can be approximated to a cylinder extending perpendicular to the center axis of the tubular body 13. At this time, when a line of intersection between an outer peripheral surface of the cylinder and the tubular body 13 is seen from the front in the direction perpendicular to the center axis of the tubular body 13, the intersection line has a circular shape as shown by a dotted line on the left side of FIG. 6. Therefore, if the distal end portion 21b of the peripheral edge of the side hole 21 is formed so as to have the semi-circular shape when projected onto the plane in the direction perpendicular to the center axis of the tubular body 15, since the medical instrument having the circular contour in cross section is brought into contact with the peripheral edge of the side hole 21 at its extreme end point on the distal side or in one continuous range including such an extreme end point as its center, it is possible to effectively provide a backup function that, while keeping the contact resistance low, prevents the medical instrument from deflecting to the lateral front side (distal side) even if a reaction force acts on the medical instrument from its tip portion when a pushing force is applied to the medical instrument. Further, if the distal end portion 21b of the peripheral edge of the side hole 21 has a semi-circular shape, the area of the peripheral wall of the tubular body 13 to be removed for forming the side hole 21 decreases compared to the case where the distal end portion 21b of the peripheral edge of the side hole 21 has a semi-elliptical shape. As a result, it is possible to reduce a reduction in the strength of the tubular body 13 in a side-hole forming region so that when an external force acts on the tubular body 13, it is possible to suppress occurrence of kinking or bending of the tubular body 13 in its side-hole forming region.

As shown in FIG. 7, at a tip portion in the inner lumen of the tubular body 13 (specifically, between the tip opening 13a and the side hole 21 in the inner lumen of the tubular body 13), a non-return valve 33 is provided as a backflow restriction mechanism for restricting backflow of a liquid (specifically, a body fluid in a tubular organ of a human body) entering from the tip opening 13a of the tubular body 13 to the side hole 21.

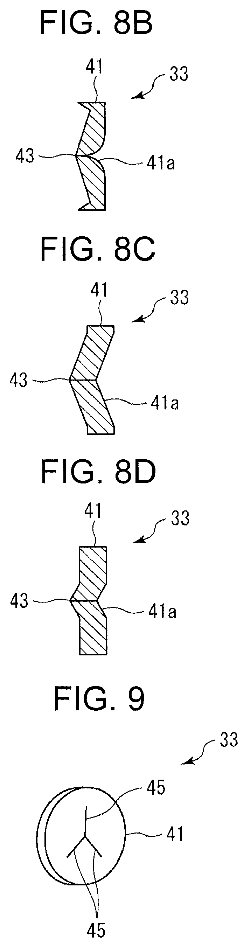

The non-return valve 33 is similar to the non-return valve of the instrument body 15 and is configured to allow insertion of an introduction assistance instrument guide wire (not shown) and to prevent passage of liquid from the tip of the tubular body 13 in the state where the introduction assistance instrument guide wire is not inserted therein. The non-return valve 33 can employ various known configurations. For example, as shown in FIG. 8A, the non-return valve 33 can be configured such that a single slit 43 is provided at a central portion of a disk body 41 formed of an elastic material. In order to effectively prevent the passage of liquid from the tip side to the base end side, portions on both sides of the slit 43 may be in contact with each other so as to protrude in a V-shape toward the tip portion of the tubular body 13, for example, as various configurations shown in section in FIGS. 8B to 8D.

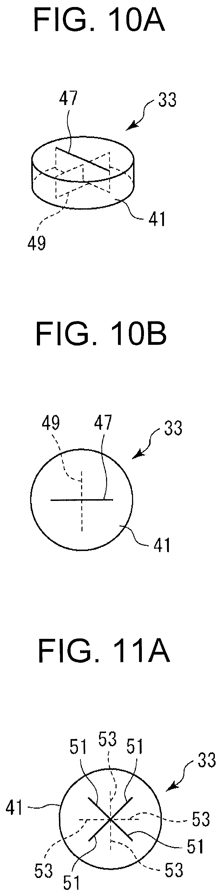

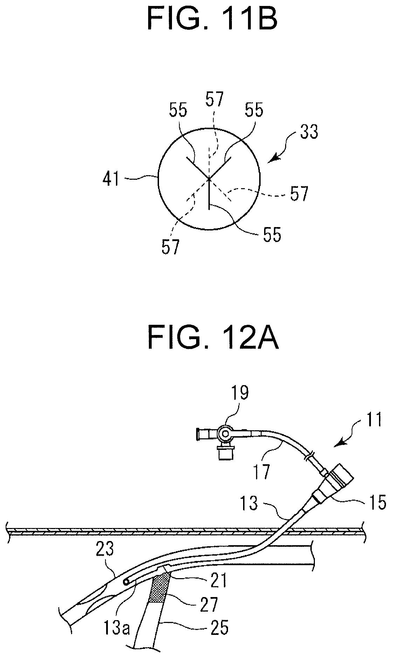

The non-return valve 33 is not limited to the embodiments shown in FIGS. 8A to 8D. For example, the single slit 43 is provided in the disk body 41 in the embodiments of FIGS. 8A to 8D, but, as shown in FIG. 9, a plurality of slits (in FIG. 9, three slits) 45 may be provided to extend radially at equal angular intervals from a center of a disk body 41. Further, as shown in FIGS. 10A and 10B, use may be made of a non-return valve 33 configured such that a slit 47 extending from one surface of a disk body 41 to a depth less than the thickness of the disk body 41 and a slit 49 extending from the other surface of the disk body 41 to a depth less than the thickness of the disk body 41 intersect each other in a cross shape inside the disk body 41. In addition, as shown in FIGS. 11A and 11B, use can also be made of a non-return valve 33 configured such that two sets of slits (four slits 51, 53 in FIG. 11A, three slits 55, 57 in FIG. 11B) extending radially at equal angular intervals from the center of a disk body 41 and each having a depth less than the thickness of the disk body 41 are, respectively, formed on one surface and the other surface of the disk 41 in such a way that the angular positions of the sets of slits are offset from each other between the opposite surfaces.

Next, referring to FIGS. 12A to 12C, a method of using the introduction assistance instrument 11 will be described using, as an example, the case where the introduction assistance instrument 11 is applied to a blood vessel of a human body.

First, an introduction assistance instrument guide wire (not shown) is inserted into a vessel (introduction vessel 23) using a puncture needle by the Seldinger technique or the like, and then the tip (distal end portion) of the introduction assistance instrument 11 is inserted to a base end portion of the introduction assistance instrument guide wire so as to insert the tubular body 13 of the introduction assistance instrument 11 into the introduction vessel 23 along the introduction assistance instrument guide wire. Further, the introduction assistance instrument 11 is placed in position in the introduction vessel 23 so that the side hole 21 of the tubular body 13 is disposed near an entrance of a side branch vessel 25 which bifurcates from the introduction vessel 23 and has a lesion site 27, and then the introduction assistance instrument guide wire is removed from the introduction assistance instrument 11 (see FIG. 12A). In this process, the non-return valve 33 provided at the tip portion of the tubular body 13 allows passage of the introduction assistance instrument guide wire, and therefore it does not prevent insertion of the introduction assistance instrument 11 to the introduction assistance instrument guide wire or the removal of the introduction assistance instrument guide wire from the introduction assistance instrument 11. Since the non-return valve 33 is provided between the tip opening and the side hole 21 in the inner lumen of the tubular body 13, in the state where the tip opening 13a of the tubular body 13 of the introduction assistance instrument 11 is located in the introduction vessel 23 while the side hole 21 is located outside the introduction vessel 23 (e.g. in a subcutaneous tissue around the vessel), blood entering the inner lumen of the tubular body 13 from the tip opening 13a can be prevented from flowing out to the outside of the introduction vessel 23 through the side hole 21. Further, since the non-return valve and the three-way switching valve are provided at the base end portion of the instrument main body 15 and at the free end portion of the connecting tube 17 in the introduction assistance instrument 11, even if the introduction assistance instrument 11 is kept placed in the introduction vessel 23 in the state where the tubular body 13 is inserted into the introduction vessel 23 so that the side hole 21 is in the introduction vessel 23, blood entering the inner lumen of the tubular body 13 from the side hole 21 can be prevented from flowing out to the outside of the human body.

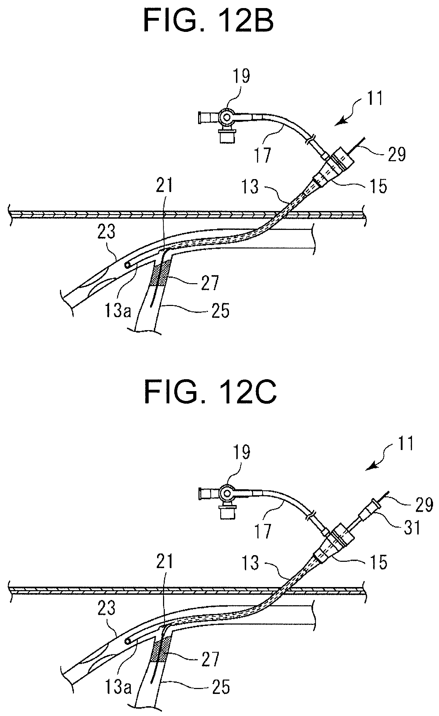

Then, as shown in FIG. 12B, a treatment instrument guide wire 29 is inserted into the inner lumen of the tubular body 13 of the introduction assistance instrument 11 through the non-return valve (not shown) provided at the base end portion of the instrument body 15 of the introduction assistance instrument 11, is extended out through the side hole 21 toward the side branch vessel 25, and then is pushed forward until its tip passes beyond the lesion site. In this process, even if the lesion site has a narrowed part and a reaction force acts on the tip portion of the treatment instrument guide wire 29, the peripheral wall of the tubular body 13 and the peripheral edge of the distal end portion 21a of the side hole 21 exhibit the backup function, and therefore it is possible to inhibit lateral deflection of a portion, near a bent portion, of the treatment instrument guide wire 29, thereby making it possible to efficiently transmit a pushing force to the tip portion of the treatment instrument guide wire 29. Thus, the treatment instrument guide wire 29 easily passes through the narrowed part.

Then, as shown in FIG. 12C, the tip of a treatment instrument 31 is inserted to a base end portion of the treatment instrument guide wire 29, thereby inserting the treatment instrument 31 into the tubular body 13 of the introduction assistance instrument 11 through the non-return valve provided at the base end portion of the instrument body 15 of the introduction assistance instrument 11. Then, the treatment instrument 31 is pushed forward in the inner lumen of the tubular body 13 of the introduction assistance instrument 11 along the treatment instrument guide wire 29 and is further guided into the side branch vessel 25 through the side hole 21, thereby treating the lesion site. Even if a reaction force acts on the treatment instrument 31 when passing through the narrowed part of the lesion site, since the peripheral wall of the tubular body 13 and the peripheral edge on the distal end portion 21a side of the side hole 21 exhibit the backup function, it is possible to inhibit lateral deflection of a portion, near a bent portion, of the treatment instrument 31 and thus it is possible to efficiently transmit a pushing force to the tip portion of the treatment instrument 31. Therefore, the treatment instrument 31 easily passes through the narrowed part.

When a lesion site is in the introduction vessel 23, the treatment instrument guide wire 29 may be passed through the non-return valve 33 and extended out from the tip opening 13a of the tubular body 13, thereby guiding the treatment instrument 31 to the lesion site along the treatment instrument guide wire 29 to treat the lesion site. That is, the introduction assistance instrument 11 can deal with both the treatment of the introduction vessel 23 and the treatment of the side branch vessel 25.

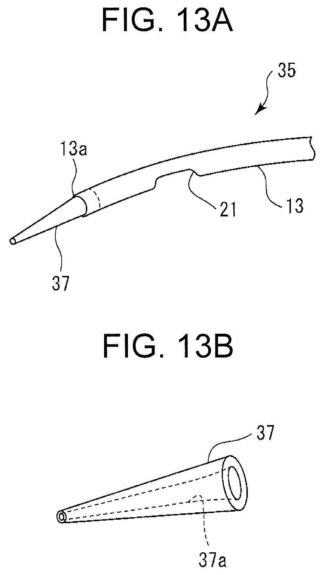

FIG. 13A shows an introduction assistance instrument 35 with a side hole according to a second embodiment of the present invention. In FIG. 13A, components common to the introduction assistance instrument 11 with the side hole of the first embodiment are denoted by the same reference signs.

The introduction assistance instrument 35 of the second embodiment differs from the introduction assistance instrument 11 of the first embodiment in that it includes, as a backflow restriction mechanism and instead of the non-return valve 33, a tip member 37 provided at the tip of a tubular body 13, and has the same configuration as the introduction assistance instrument 11 of the first embodiment in the other points. Therefore, herein, a description of the configuration common to the introduction assistance instrument 11 of the first embodiment will be omitted and only the tip member 37 will be described.

The tip member 37 is formed of a synthetic resin material and has a shape such that its outer peripheral surface tapers toward the tip side. As shown in FIG. 13B on an enlarged scale, in order to allow an introduction assistance instrument guide wire (not shown) to pass through, a through hole 37a having a diameter smaller than the diameter of an inner lumen of the tubular body 13 and slightly greater than the diameter of the introduction assistance instrument guide wire is formed at a central portion of the tip member 37. Preferably, as shown in FIG. 13B, the through hole 37a has a tapered shape in which a cross section of a distal end portion (an end portion on the free end side) is smaller than a cross section of a proximal end portion (an end portion on the side connected to a tip opening 13). The tip member 37 may be fixed to the tip opening 13a of the tubular body 13 using an adhesive or the like, or may be formed integrally with the tubular body 13.

Since the tip member 37 is formed therein with the through hole 37a that allows passage of the introduction assistance instrument guide wire, even if the tip member 37 is provided at the tip portion of the tubular body 13, insertion of the introduction assistance instrument 35 to the introduction assistance instrument guide wire is not prevented. Further, with the introduction assistance instrument 35, since a treatment instrument guide wire and a treatment instrument are extended out to the outside through a side hole 21, it is not necessary to extend out the treatment instrument guide wire or the treatment instrument through the through hole 37a of the tip member 37. Therefore, if the diameter of a tip opening of the through hole 37a is determined to a minimum size that allows passage of the introduction assistance instrument guide wire, it is possible to minimize entry of blood into the inner lumen of the introduction assistance instrument 35. As a result, in the state where the tip of the tubular body 13 of the introduction assistance instrument 35 is located in an introduction vessel 23 while the side hole 21 is located outside the introduction vessel 23 (e.g. in a subcutaneous tissue around the vessel), it is possible to minimize the amount of blood that enters the inner lumen from the tip of the tubular body 13 and flows back to the outside of the introduction vessel 23 through the side hole 21. Further, since the tip member 37 has the shape in which its outer peripheral surface tapers toward the tip side, an effect of facilitating insertion of the introduction assistance instrument 35 into a puncture hole of the introduction vessel 23 and expansion of the puncture hole is also exhibited.

While the introduction assistance instrument with the side hole according to the present invention has been described with reference to the illustrated embodiments, the present invention is not limited to the illustrated embodiments. For example, the introduction assistance instrument 11 or 35 is shown to include only one of the non-return valve 33 and the tip member 37 as the backflow restriction mechanism in the illustrated embodiments, but may include both the non-return valve 33 and the tip member 37.

### Reference Signs List

- 11: Introduction assistance instrument
- 13: Tubular body
- 15: Instrument body
- 17: Connecting tube
- 19: Three-way switching valve
- 21: Side hole
- 33: Non return valve
- 35: Introduction assistance instrument
- 37: Tip member
- 41: Disk body
- 43: Slit
- 45: Slit
- 47: Slit
- 49: Slit
- 51: Slit
- 53: Slit
- 55: Slit
- 57: Slit

## Claims

1. An introduction assistance instrument with a side hole, comprising a tubular body adapted to be inserted into a tubular organ of a human body, said side hole formed in a peripheral wall of the tubular body so as to allow a medical instrument to be introduced into the tubular organ through the side hole of the tubular body,
**characterized by** comprising a backflow restriction mechanism provided at a tip portion of the tubular body, said backflow restriction mechanism restricting backflow of a body fluid in the tubular organ from a tip opening of the tubular body to the side hole.

2. The introduction assistance instrument according to claim 1, wherein the backflow restriction mechanism comprises a non-return valve provided between the tip opening of the tubular body and the side hole in an inner lumen of the tubular body.

3. The introduction assistance instrument according to claim 1, wherein the backflow restriction mechanism comprises a tip member provided at a tip of the tubular body and shaped so that an outer peripheral surface thereof tapers toward a tip side thereof, said tip member formed at a center portion thereof with a through hole that allows passage of a guide wire.

4. The introduction assistance instrument according to claim 3, wherein a cross section of a distal end portion of the through hole is smaller than a cross section of a proximal end portion of the through hole.

5. The introduction assistance instrument according to claim 1, wherein the side hole has a peripheral edge including a distal end portion formed so as to have a semi-circular shape when projected onto a plane and a proximal end portion formed so as to have a semi-elliptical shape when projected onto the plane.

6. The introduction assistance instrument according to claim 2, wherein a peripheral edge of the side hole includes a distal end portion formed so as to have a semi-circular shape when projected onto a plane and a proximal end portion formed so as to have a semi-elliptical shape when projected onto the plane.

7. The introduction assistance instrument according to claim 3, wherein the side hole has a peripheral edge including a distal end portion formed so as to have a semi-circular shape when projected onto a plane and a proximal end portion formed so as to have a semi-elliptical shape when projected onto the plane.

8. The introduction assistance instrument according to claim 4, wherein the side hole has a peripheral edge including a distal end portion formed so as to have a semi-circular shape when projected onto a plane and a proximal end portion formed so as to have a semi-elliptical shape when projected onto the plane.

9. The introduction assistance instrument according to any one of claims 1 to 8, wherein the introduction assistance instrument comprises a sheath introducer, a guide catheter, or a catheter for penetrating a stenotic lesion.
